# EUROPEAN PATENT APPLICATION

(11) **EP 4 635 522 A1**
(43) Date of publication of application: **22.10.2025**
(21) Application number: 25170852.5
(22) Date of filing: 15.04.2025
(51) Int. Cl.: A61L 26/00, A61K 8/87, A61K 8/891, A61Q 19/00

(54) **PHARMACEUTICAL COMPOSITION FOR PREVENTING OF ABNORMAL SCARS GROWTH AND TREATMENT OF SCARS**

(30) Priority: 15.04.2024 EP 24170376
(71) Applicant: EmergoPharm Sp. z.o.o. Sp.K., 02-777 Warszawa (PL)
(72) Inventor: Cieciara, Mariusz Radoslaw, 05-520 Konstancin-Jeziorna (PL); Roman, Beata, 02-759 Warszawa (PL)
(74) Representative: JD&P Patent Attorneys Joanna Dargiewicz & Partners

(57) **Abstract**

The object of the present invention is a pharmaceutical composition for use in the prevention and treatment of scars containing silicones in the amount of 95 to 99.99% by weight and the amount of 0.01 to 5% by weight of an aqueous dispersion of a polymer based on ethoxylated fatty alcohol urethane, containing 19 to 21% by weight of polyurethane.

## Description

### Technical field

The present invention relates to a pharmaceutical composition for use in the prevention of abnormal scar formation and in the treatment of scars, which is an intermediate form between patches and silicone-based gels.

### Background

A scar forms during the wound healing process when tissue damaged during injury is replaced by well-vascularized connective (granulation) tissue. This process can be divided into three stages: wound cleansing (the inflammatory phase, during which necrotic tissue and microorganisms are removed from the wound), granulation (proliferation), and epithelialization, which is the formation of new epithelium. [Zurada J. M., Kriegel D., Davis C. D.: Topical treatments for hypertrophic scars. J. Am. Acad. Dermatol. 2006, 55: 1024-1031]. Proper healing requires increased activity and migration of fibroblasts into the wound bed, as well as a correct collagen cross-linking process, during which collagen undergoes polymerization in the extracellular space, forming bundles of insoluble fibrils. [Chipev C. C., Simon M.: Phenotypic differences between dermal fibroblasts from different body sites determine their responses to tension and TGF-beta1. BMC Dermatol. 2002, 2: 13-17].

Most scars heal spontaneously and do not require specialized treatment. However, in some cases, abnormal scarring process may occur, resulting in hypertrophic scars or keloids. Abnormal scars pose a significant problem for a patient-not only aesthetically, but also medically-as they can lead to contractures, pain, and itching.

Due to the lack of universally effective therapies and the challenges associated with treating of already existing scar, preventive measures are recommended, especially for individuals prone to abnormal scarring. Such measures involve the earliest possible use of non-invasive therapeutic methods aimed at preventing the formation of abnormal scar.

Silicone gel has been used in the treatment of scars since 1980. Silicone-based products have been recognized as first-line therapy, the gold standard in scar treatment, and have demonstrated efficacy in both the prevention and treatment of pathological scars.

According to a Cochrane meta-analysis performed by O'Brien and Jones, silicone gel as a means of preventing pathological scars was effective in high-risk patients, and improvements in scar thickness and scar color were also reported in patients, using silicone gel to treat pathological scars.

The efficacy and safety of this gold standard non-invasive therapy have been demonstrated in multiple clinical trials.

Excessive scarring can have unpleasant physical, aesthetic, psychological and social consequences. Physical symptoms may include itching, stiffness, scar contracture, tenderness and pain.

When treating convex scars, any method that reduces excessive superficial tissue growth is used. The most commonly used include silicone gels and patches and steroid therapy.

Polysiloxane-based preparations are available over the counter and very rarely cause adverse effects. The mechanism of their action is not completely known. Scar reduction is thought to occur through hydration and occlusion of the scar tissue. Other theories assume a function of oxygen reduction in the scar, a chemical action of silicone oils or a change in skin potential. Gels are recommended for skin lesions located, for example, in skin folds or on noticeable areas of the body, such as the face, while silicone patches are recommended for flat and extensive areas.
1. Monstrey S, Middelkoop E, Vranckx JJ, Bassetto F, Ziegler UE, Meaume S, Téot L. Updated scar management practical guidelines: non-invasive and invasive measures. J Plast Reconstr Aesthet Surg. 2014 Aug;67(8):1017-25. doi: 10.1016/j.bjps.2014.04.011. Epub 2014 May 14. PMID: 24888226.
2. Tian F, Jiang Q, Chen J, Liu Z. Silicone gel sheeting for treating keloid scars. Cochrane Database Syst Rev. 2023 Jan 3;1(1):CD013878. doi: 10.1002/14651858.CD013878.pub2. PMID: 36594476; PMCID: PMC9808890. https://www.ncbi.nlm.nih.gov/pmc/articles/PMC9808890/pdf/CD013878.pdf
3. Wang F, Li X, Wang X, Jiang X. Efficacy of topical silicone gel in scar management: A systematic review and meta-analysis of randomised controlled trials. Int Wound J. 2020 Jun;17(3):765-773. doi: 10.1111/iwj.13337. Epub 2020 Mar 2. PMID: 32119763; PMCID: PMC7949016.
4. Marzena Jamrógiewicz, Maria Zebrowska, Jerzy 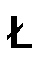 ukasiak, Matgorzata Sznitowska, Silikonowe preparaty do leczenia powierzchniowego blizn,
   https://www.ptfarm.pl/pub/File/Farmacja%20Polska/2010/06-2010/10%20%20Silikonowe%20preparaty.pdf
5. Marlena Przew 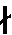 ocka-G 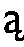 ga 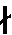 a, Wspo 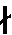 czesny model post 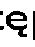 powania z problemem blizn w kosmetologii i medycynie estetycznej.
   http://aestheticcosmetology.com/wp-content/uploads/2021/02/acm-2021-01-przewlocka-pl.pdf

Silicone gels are preparations characterized by a semi-solid consistency and varying viscosity. Once the gel is applied, an elastic and transparent layer is formed on the surface of the skin.

Silicone gels are more popular than silicone patches due to their unlimited applicability to different parts of the body, but also because of their availability and much lower price compared to patches, which also often peel off and then fail to perform their function.

Other occlusive treatments are also effective, however the degree of occlusion is important, as complete occlusion results in excessive hydration with effects on surface bacterial levels and undesirable physical changes in the epithelium.

Polysiloxanes are polymers whose main chain is made up of alternating silicon and oxygen atoms. Silicones in a gel form applied to the skin increase the hydration of the stratum corneum. By optimizing the hydration of the epidermis, they create the right conditions for proper scar maturation.

Diffusion of water through the stratum corneum occurs, and is greatly increased in pathological situations such as scars, so it is important to use appropriate preparations to ensure adequate hydration and occlusion of the scar tissue.
6.Thomas A. Mustoe Silicone Gel for Scar Prevention; Textbook on Scar Management, 2020 https://link.springer.com/chapter/10.1007/978-3-030-44766-3_23

The skin acts as a barrier to prevent water evaporation from internal tissues. The skin's water content helps maintain its function.

Transepidermal water loss is referred to by the term TEWL (transepidermal water loss), meaning the outward diffusion of water through the skin. TEWL measurements are used to assess the skin's water barrier function. An increase in TEWL reflects a deterioration of the water barrier.

The results of these measurements are a good indicator of the skin barrier restoration and a useful indicator of the scar maturation process.

Results of TEWL or transepidermal water loss or skin hydration measurements should be reported as differences or percentage change rather than absolute values. This approach partially eliminates influence of other factors.

The aim of the present invention was to develop a pharmaceutical composition for use in the prevention of abnormal scar formation and in the treatment of scars, with a consistency that allows good spreading anywhere on the surface of a skin, forming a flexible, invisible film that maintains an adequate degree of skin hydration, and protects the scar from external factors, preferably, providing a non-sticky and matte finish.

This purpose has been achieved by the pharmaceutical composition according to the present invention.

### Summary

Thus, the object of the present invention is a pharmaceutical composition for use in the prevention of abnormal scar formation and in the treatment of scars, comprising:
- silicones in the amount of 95 to 99.99% by weight, and
- 0.01 to 5% by weight of an aqueous dispersion of a polymer based on ethoxylated fatty alcohol urethane, containing 19 to 21% by weight of polyurethane,
wherein as silicone it contains dimethicone and cross-linked dimethicone with a high viscosity of 300,000 to 600,000 mm²/s at 25°C and dimethicone and cross-linked dimethicone with a low viscosity of 3000 to 6000 mPa.s (3260-6315 mm²/s) at 25°C.

Preferably, the pharmaceutical composition contains dimethicone and cross-linked dimethicone with a high viscosity of 300,000 to 600,000 mm²/s at 25°C, in an amount of 60 to 80% by weight, more preferably 65 to 75% by weight and dimethicone and cross-linked dimethicone with a low viscosity of 3000 to 6000 mPa.s (3260-6315 mm²/s) at 25°C in the amount of 20 to 40% by weight, more preferably 25 to 35% by weight.

Preferably, the pharmaceutical composition contains dimethicone and cross-linked dimethicone with a high viscosity of 300,000 to 600,000 mm²/s at 25°C in the amount of 68 to 72% by weight, and dimethicone and cross-linked dimethicone with a low viscosity of 3000 to 6000 mPa.s (3260-6315 mm²/s) at 25°C in the amount of 28 to 32% by weight.

Preferably, the pharmaceutical composition contains 0.02 to 1% by weight, preferably 0.02 to 0.2% by weight of an aqueous dispersion of a polymer based on ethoxylated fatty alcohol urethane, containing 19 to 21% by weight of polyurethane.

Preferably, the pharmaceutical composition contains dimethicone and cross-linked dimethicone with a high viscosity of 300,000 to 600,000 mm²/s at 25°C in the amount of 69.95 by weight, and dimethicone and cross-linked dimethicone with a low viscosity of 3000 to 6000 mPa.s (3260-6315 mm²/s) at 25°C in the amount of 29.95% by weight and 0.1% by weight of an aqueous dispersion of a polymer based on ethoxylated fatty alcohol urethane, containing 19 to 21% by weight of polyurethane.

Preferably, the pharmaceutical composition contains water in the amount of 0.07% to 8% by weight.

Preferably, the pharmaceutical composition contains up to 0.15% by weight of additives.

Preferably, the aqueous dispersion of the polymer based on ethoxylated fatty alcohol urethane contains Polyurethane-39.

Another subject of the present invention is a composition comprising:
- silicones in an amount of 95 to 99.99% by weight, and
- 0.01 to 5% by weight of an aqueous dispersion of a polymer based on ethoxylated fatty alcohol urethane containing 19 to 21% by weight of polyurethane,
wherein as silicone it contains dimethicone and cross-linked dimethicone with a high viscosity of 300,000 to 600,000 mm²/s at 25°C, and dimethicone and cross-linked dimethicone with low viscosity of 3000 to 6000 mPa·s (3260-6315 mm²/s) at 25°C.

Preferably, the composition contains dimethicone and cross-linked dimethicone with high viscosity of 300,000 to 600,000 mm²/s at 25°C in an amount of 60 to 80% by weight, and dimethicone and cross-linked dimethicone with low viscosity of 3000 to 6000 mPa·s (3260-6315 mm²/s) at 25°C in an amount of 20 to 40% by weight.

Preferably, the composition contains dimethicone and cross-linked dimethicone with high viscosity of 300,000 to 600,000 mm²/s at 25°C in an amount of 65 to 75% by weight, and dimethicone and cross-linked dimethicone with low viscosity of 3000 to 6000 mPa·s (3260-6315 mm²/s) at 25°C in an amount of 25 to 35% by weight.

Preferably, the composition contains dimethicone and cross-linked dimethicone with high viscosity of 300,000 to 600,000 mm²/s at 25°C in an amount of 68 to 72% by weight, and dimethicone and cross-

linked dimethicone with low viscosity of 3000 to 6000 mPa·s (3260-6315 mm²/s) at 25°C in an amount of 28 to 32% by weight.

Preferably, the composition contains 0.02 to 1% by weight, preferably 0.02 to 0.2% by weight, of an aqueous dispersion of a polymer based on ethoxylated fatty alcohol urethane containing 19 to 21% by weight of polyurethane.

Preferably, the composition contains dimethicone and cross-linked dimethicone with high viscosity of 300,000 to 600,000 mm²/s at 25°C in an amount of 69.95% by weight, and dimethicone and cross-linked dimethicone with low viscosity of 3000 to 6000 mPa·s (3260-6315 mm²/s) at 25°C in an amount of 29.95% by weight, as well as 0.1% by weight of an aqueous dispersion of a polymer based on ethoxylated fatty alcohol urethane containing 19 to 21% by weight of polyurethane.

Another aspect of the invention is the use of the above-defined composition for the prevention of scar formation and for the treatment of scars.

### Detailed description of the invention

The present invention consists in combination of ingredients in a gel form: silicones, such as dimethicone and/or cross-linked dimethicone (INCI: Dimethicone (and) Dimethicone Crosspolymer) and polyurethane (e.g. in the form of POLYURETHANE-39), in order to obtain a formulation that stays on the skin longer and protects the skin better, against excessive water evaporation, than standard silicone gels without polyurethane.

This combination ensures adequate hydration of the skin for a longer period of time, which is extremely important in the treatment of scars, especially in those areas where the use of patches is problematic or impossible.

The composition also does not aim for total occlusion, resulting in excessive hydration, with effects on surface bacterial levels and undesirable physical changes to the epithelium.

The aim of the composition was to obtain a product with a consistency that allows good spreading of the product in problematic areas, creating a flexible, invisible film on the skin surface that maintains an adequate level of skin hydration and protects the scar from external factors and gives a less sticky and matte finish.

An unexpected result of using the substance POLYURETHANE-39 is obtaining an additional function than that declared for this ingredient, i.e. regulating the consistency and influencing the viscosity of the product.

### https://cosmileeurope.eu/pl/inci/szczegoly/12532/polyurethane-39/

The unique combination of the composition ingredients, i.e. silicones with polyurethane in a gel form, for the treatment of scars, has not been used before.

The combination of these ingredients in a gel form made it possible to create a product that is an intermediate form between patches and gels. Its uniqueness is the longer scar protection, compared to silicone gels without polyurethanes, and the possibility of using it in areas where patches cannot be used.

Without being bound by any theory, the investors suspect that the polyurethane creates a network, reinforcing the structure of the silicone gel, contributing to the creation of an intermediate product between a patch with a stiffened polyurethane base and a flowing and easily abradable silicone gel. This results in a product, that covers the skin with a thin film, is matte and invisible, and lasts until washed off with water and detergent, while maintaining adequate hydration of the scar, promoting faster healing.

In addition, it has been found that the composition according to the present invention is stable and retains its properties, such as consistency, transparency during long storage, which is a problem in many preparations on the market, which after opening spill out of the packaging and liquid can be observed on the surface or even stratification of the product into two phases.

Providing a suitable environment and protective barrier is essential for proper scar management to avoid abnormal scar formation.

An object of the present invention is a pharmaceutical composition having the composition shown in Table 1:

**Table 1**

| **Substance** | **Content [% by weight]** |
|---|---|
| Silicones - dimethicone and cross-linked dimethicone with a high viscosity of 300,000 to 600,000 mm²/s at 25°C | 60-80 |
| INCI Dimethicone (and) Dimethicone Crosspolymer | |
| Silicones - dimethicone and cross-linked dimethicone with a low viscosity of 3000 to 6000 mPa.s at 25°C | 20-40 |
| INCI Dimethicone (and) Dimethicone Crosspolymer | |
| Polyurethanes in the form of an aqueous polymer dispersion based on ethoxylated fatty alcohol urethane, comprising Polyurethane-39 (19-21%), water (70-80%), excipients (max. 1.2%) with a viscosity of 40 to 100 Pa.s at 23°C | 0.01-5 |

### EXAMPLES

### Example 1

### Method for preparing the pharmaceutical composition according to the present invention

The ingredients were weighed according to the amounts given in the formulation (for example in Table 2). Then, polyurethane in the form of an aqueous polymer dispersion based on ethoxylated fatty alcohol urethane, comprising Polyurethane-39 (19-21%), water (70-80%), additives (max. 1.2%), was added to the part of the lower viscosity silicones constituting about 30% of the composition, and combined together using a pneumatic stirrer set at 3.5-4.5 bar. The remaining high viscosity silicones were then added in portions. The mixture was stirred until a homogeneous mass was obtained for approximately 1 hour. The pneumatic stirrer was turned off and the setting checked for lumps. If lumps are present, the mixing should be extended by 15 minutes. A quality check of the batch produced was carried out. It was also observed that the order of raw materials added can be interchangeable for this composition and gave an equally stable product.

### Example 2

### Measurement of TEWL values

By the method according to Example 1, two formulations were prepared with the compositions given in Table 2:

**Table 2**

| **Raw material** | **Product S** | **Product S+P** |
|---|---|---|
| | **amount [% by weight]** | **amount [% by weight]** |
| Silicones - dimethicone and cross-linked dimethicone with a high viscosity of 350,000 to 575,000 mm²/s INCI Dimethicone (and) Dimethicone Crosspolymer DOWSIL^{™} EL-9140 DM Silicone Elastomer Blend, manufacturer: DOW, kinematic viscosity 350,000-575,000 mm²/s (HELIPATH RV TD, Spindle 94 @ 2.5 RPM, CTM0050 method, at 25°C) | 70 | 69.95 |
| Silicones - dimethicone and cross-linked dimethicone with a low viscosity of 3000 to 6000 mPa.s (3260-6315 mm²/s) | 30 | 29.95 |
| INCI Dimethicone (and) Dimethicone Crosspolymer RJS-1209, manufacturer: RUISiL (kinematic viscosity (mm²/s) (@25°C, 4#, 60 rpm, mPa•S): 3000~6000 | | |
| Polyurethanes in the form of an aqueous polymer dispersion based on ethoxylated fatty alcohol urethane, comprising Polyurethane-39 (19-21%), water (70-80%), excipients (max. 1.2%) with a viscosity of 40 to 100 Pa.s | | 0.100 |
| INCI: Polyurethane-39 | | |
| Luvigel^{®} Star AT 3, manufacturer: BASF | | |
| Kinematic viscosity: ca. 97,000 mm²/s (23°C) (OECD 114) and ca. 37,000 mm²/s (40°C) (OECD 114) | | |
| Dynamic viscosity: ca. 100,000 mPa.s (23°C) (DIN 53018), manufacturer: BASF (Brookfield viscosity (as is; sp. 7, 40 rpm, RVT; 23°C), 40-100 Pa.s, ISO 2555 | | |

**Table 3**

| **Qualitative and quantitative composition of the product S+P** | | |
|---|---|---|
| 1. | DOWSIL EL-9140 DM SILICONE ELASTOMER BLEND | 69.95% |
| | Dimethicone (and) Dimethicone Crosspolymer | |
| 2. | RJS-1209 | 29.95% |
| | Dimethicone (and) Dimethicone Crosspolymer | |
| 3. | LUVIGEL STAR AT 3 | 0.10% |
| | Polyurethane-39 | |

**Table 4**

| **Physicochemical properties of the product S+P** | | | |
|---|---|---|---|
| Nr | Parameter | Method | Requirements |
| 1. | Form | IS-11/03 | Homogeneous, clear to slightly opaque gel |
| 2. | Color | IS-11/03 | Colorless |
| 3. | Odor | IS-11/03 | Inherent to the raw materials used |
| 4. | Net weight [g] | IS-11/03 | 15 +/- |
| 5. | Density at 20°C, [g/ml] | IS-11/03 | 0.85-0.90 g/cm³ |
| 6. | Viscosity at 20°C | IS-11/03 | 15,000-45,000 cP |

**Table 5**

| **Microbiological profile of the product S+P** | | | |
|---|---|---|---|
| **Microbiological testing in accordance with the requirements of the current edition of the Polish Pharmacopoeia, for dermal application.** | | | |
| Nr | Parameter | Method | Requirements |
| 1 | Aerobic bacterial count | in accordance with the current edition of the Polish Pharmacopoeia | ≤ 2x 10² CFU/g |
| 2 | Yeast and mold count | in accordance with the current edition of the Polish Pharmacopoeia | ≤ 2x 10¹ CFU/g |
| 3 | *Staphylococcus aureus* | in accordance with the current edition of the Polish Pharmacopoeia | none in 1 g |
| 4 | *Pseudomonas aeruginosa* | in accordance with the current edition of the Polish Pharmacopoeia | none in 1 g |

Preliminary tests using the formulations with the above compositions have shown that adding the raw material containing Polyurethane-39 makes it possible to obtain a higher TEWL, and thus the skin is better protected and the product has better and longer effects.

Transepidermal water loss testing was performed using a laboratory skin testing device from Courage + Khazaka electronic GmbH and a Tewameter probe.

Measurements were made before the gels were applied, after the gels were applied and 5 hours after the gels were applied according to the following procedure:
- the probe should be placed in the correct position on the skin, so that it adheres to it but is not pressed;
- press the button on the probe housing and observe the results, wait until the reading stabilizes;
- end the measurement by pressing the button on the probe housing again.

Table 6 below summarizes the absolute TEWL values obtained, and what is important is the TEWL difference. The greater the difference, the better the skin is protected and hydrated.

It is important to bear in mind that in the case of scars where TEWL is higher than in healthy skin, we are concerned with applying an adequate semi-permeable barrier to restore adequate hydration.

It should be borne in mind, that in the case of scars where the TEWL is higher than in the case of healthy skin, we are concerned with applying an appropriate semi-permeable barrier to restore adequate hydration.

**Table 6**

| Person | Skin before application | | Gel after application | | Difference | | Gel after 5h from application | | Difference | |
|---|---|---|---|---|---|---|---|---|---|---|
| | **S** | **S+P** | **S** | **S+P** | **S** | **S+P** | **S** | **S+P** | **S** | **S+P** |
| Average TEWL reduction | 9.4 | 11.6 | 9.3 | 6.5 | **-0.1** | **-5.1** | 7.7 | 8.6 | **-1.6** | **-3.0** |
| TEWL reduction [%] | | | | | **1.1** | **44.0** | | | **17.4** | **25.6** |

The results illustrate that Product S+P containing dimethicone and cross-linked dimethicone and the raw material with Polyurethane-39, gave better results, both after application and after 5 hours, compared to Product S containing only dimethicone and cross-linked dimethicone.

The greater the difference in TEWL, the better the skin is protected and hydrated over a longer period of time, which is particularly important in the treatment of scars.

Additional studies were also performed on a different group of volunteers and with higher concentrations of the raw material containing Polyurethane-39, i.e. 1 %, 5 % and 10 %, and transepidermal water loss (TEWL) was assessed. The obtained results show that increasing the amount of the raw material containing Polyurethane-39 above 5% does not provide better results in terms of skin protection and hydration.

**Table 7**

| Scope of the Study | Study result | |
|---|---|---|
| | % change in TEWL after gel application compared to the skin without product | % change in TEWL after 5 h compared to the skin without product |
| *S* | *1.3%* | *7.9 %* |
| S + P 0.1% | *27.1%* | *14.2%* |
| S + P 1% | *27.3%* | *14.9%* |
| S + P 5% | *34.1%* | *19.5%* |
| S + P 10% | *32.1%* | *18.9%* |

It was observed that increasing the amount of polyurethane causes deterioration of sensory parameters. Products with higher concentrations than 1% of the raw material containing Polyurethane-39 left a sticky film on the skin surface after 5 hours.

### Example 3

### Study of a pharmaceutical composition in form of gel containing silicones and polyurethane (Table 2, Product S+P)

Preliminary test results demonstrate the product's effectiveness in preventing the formation of abnormal scars, as well as in scar treatment, and improving their appearance.

The study is conducted under the supervision of a dermatologist. The preparation is applied to newly formed scars, starting from 2 months after complete wound healing, up to 4 years post scar formation. The treated scars include postoperative, burn-related, and traumatic scars located on the abdomen, arm, knee, torso, and upper body.

Scar parameters were assessed using the Patient Scar Assessment Scale (PSAS) and the Observer Scar Assessment Scale (OSAS), which together form the Patient and Observer Scar Assessment Scale (POSAS).

Preliminary Effects of the Preparation Based on Investigator Assessment Using the POSAS Scale According to the POSAS-based investigator's evaluation, the preparation-when used regularly-in the preliminary analysis has shown the following preliminary effects:
visibly reduces skin redness
provides a sensation of increased scar elasticity
visibly flattens the scar
reduces the feeling of discomfort or skin tightness
alleviates itching
visibly lightens the scar
visibly smoothens the scar
visibly improves the overall appearance of the scar.

The overall assessment of scar appearance improvement met the expectations of the majority of study participants. The tested preparation is well tolerated at the site of application. The composition remains on the skin longer than other previously used scar products in the form of creams or gels, which is particularly important during the healing and care process. Additionally, the composition adheres to the skin longer than silicone patches and can be applied to areas where such patches couldn't be attached or places exposed to factors which promote patch detachment such as skin folds or highly mobile regions.

The composition is also virtually invisible on the skin, does not leave a greasy residue, and provides a matte finish, which may be especially beneficial for visible scars.

The composition does not require the use of additional skin adhesives, thereby reducing the risk of undesirable allergic reactions.

## Claims

1. A pharmaceutical composition comprising:
- silicones in the amount of 95 to 99.99% by weight and
- 0.01 to 5% by weight of aqueous dispersion of a polymer based on ethoxylated fatty alcohol urethane containing 19-21% by weight of polyurethane,
for use in the prevention of abnormal scar formation and in the treatment of scars,
wherein as silicone it contains dimethicone and cross-linked dimethicone, with a high viscosity of 300,000 to 600,000 mm²/s at 25°C and dimethicone and cross-linked dimethicone with a low viscosity of 3000 to 6000 mPa.s (3260-6315 mm²/s) at 25°C.

2. The pharmaceutical composition according to claim 1, **characterized in that** it contains dimethicone and cross-linked dimethicone with a high viscosity of 300,000 to 600,000 mm²/s at 25°C in the amount of 60 to 80% by weight, preferably 65 do 75% by weight and dimethicone and cross-linked dimethicone with a low viscosity of 3000 to 6000 mPa.s (3260 -6315 mm²/s) at 25°C in the amount of 20 to 40% by weight, preferably 25 to 35% by weight.

3. The pharmaceutical composition according to any of claims 1 or 2, **characterized in that** it contains dimethicone and cross-linked dimethicone with high viscosity of 300,000 to 600,000 mm²/s at 25°C in an amount of 68 to 72% by weight, and dimethicone and cross-linked dimethicone with low viscosity of 3000 to 6000 mPa·s (3260-6315 mm²/s) at 25°C in an amount of 28 to 32% by weight.

4. The pharmaceutical composition according to any of claims 1 to 3, **characterized in that** it contains 0.02 to 1% by weight, preferably 0.02 to 0.2% by weight, of an aqueous dispersion of a polymer based on ethoxylated fatty alcohol urethane, containing 19 to 21% by weight of polyurethane.

5. The pharmaceutical composition according to any of claims 1 to 4, **characterized in that** it contains dimethicone and cross-linked dimethicone with a high viscosity of 300,000 to 600,000 mm²/s at 25°C in the amount of 69.95% by weight and dimethicone and cross-linked dimethicone with a low viscosity of 3000 to 6000 mPa.s (3260-6315 mm²/s) at 25°C in the amount of 29.95% by weight and 0.1% by weight of an aqueous dispersion of a polymer based on ethoxylated fatty alcohol urethane, containing 19 to 21% by weight of polyurethane.

6. The pharmaceutical composition according to any of claims 1 to 5, **characterized in that** it contains water in the amount of 0.07% to 8% by weight.

7. The pharmaceutical composition of any of claims 1 to 6, **characterized in that** it contains additives in the amount of up to 0.15% by weight.

8. The pharmaceutical composition according to any of claims 1 to 7, **characterized in that** aqueous dispersion of a polymer based on ethoxylated fatty alcohol urethane contains Polyurethane-39.

9. A composition containing:
- silicones in an amount of 95 to 99.99% by weight, and
- 0.01 to 5% by weight of an aqueous dispersion of a polymer based on ethoxylated fatty alcohol urethane, containing 19 to 21% by weight of polyurethane,
wherein as the silicone it comprises dimethicone and cross-linked dimethicone with high viscosity of 300,000 to 600,000 mm²/s at 25°C, and dimethicone and cross-linked dimethicone with low viscosity of 3000 to 6000 mPa·s (3260-6315 mm²/s) at 25°C.

10. The composition according to claim 9, **characterized in that** it comprises dimethicone and cross-linked dimethicone with high viscosity of 300,000 to 600,000 mm²/s at 25°C, in an amount of 60 to 80% by weight, and dimethicone and cross-linked dimethicone with low viscosity of 3000 to 6000 mPa·s (3260-6315 mm²/s) at 25°C, in an amount of 20 to 40% by weight.

11. The composition according to any of claims 9 or 10, **characterized in that** it comprises dimethicone and cross-linked dimethicone with high viscosity of 300,000 to 600,000 mm²/s at 25°C, in an amount of 65 to 75% by weight, and dimethicone and cross-linked dimethicone with low viscosity of 3000 to 6000 mPa·s (3260-6315 mm²/s) at 25°C, in an amount of 25 to 35% by weight.

12. The composition according to any of claims 9 to 11, **characterized in that** it comprises dimethicone and cross-linked dimethicone with high viscosity of 300,000 to 600,000 mm²/s at 25°C, in an amount of 68 to 72% by weight, and dimethicone and cross-linked dimethicone with low viscosity of 3000 to 6000 mPa·s (3260-6315 mm²/s) at 25°C, in an amount of 28 to 32% by weight.

13. The composition according to any of claims 9 to 12, **characterized in that** it comprises 0.02 to 1% by weight, preferably 0.02 to 0.2% by weight, of an aqueous dispersion of a polymer based on ethoxylated fatty alcohol urethane, containing 19 to 21% by weight of polyurethane.

14. The composition according to any of claims 9 to 13, **characterized in that** it comprises 69.95% by weight of dimethicone and cross-linked dimethicone with high viscosity of 300,000 to 600,000 mm²/s at 25°C, 29.95% by weight of dimethicone and cross-linked dimethicone with low viscosity of 3000 to 6000 mPa·s (3260-6315 mm²/s) at 25°C, and 0.1% by weight of an aqueous dispersion of a polymer based on ethoxylated fatty alcohol urethane, containing 19 to 21% by weight of polyurethane.

15. Use of the composition as defined in any of claims 10 to 14 for preventing the formation of scars and for the treatment of scars.
